# EUROPEAN PATENT APPLICATION

(11) **EP 2 599 474 A1**
(43) Date of publication of application: **05.06.2013**
(21) Application number: 11009473.7
(22) Date of filing: 30.11.2011
(51) Int. Cl.: A61K 8/36, A61K 31/192, A61Q 7/00, A61Q 19/00

(54) **4-phenylbutyric acid for cosmetic use**

(71) Applicant: Lunamed AG, 7000 Chur (CH)
(72) Inventor: Truog, Peter, 7000 Chur (CH); Buschmann, Helmut H., 52076 Aachen (Walheim) (DE)
(74) Representative: Peters, Hajo

(57) **Abstract**

The present invention relates to a cosmetic use of a phenyl-butyric acid, a derivative or a physiologically acceptable salt thereof, like 4-phenylbutyric acid (4-PB) or its sodium salt. Particularly, the present invention provides cosmetic articles comprising phenyl-butyric acid, a derivative or a physiologically acceptable salt thereof, like 4-phenylbutyric acid (4-PB) or its sodium salt, especially at a low daily dosis for cosmetic use for treatment of various cosmetic problems of body surfaces such as hair, skin etc..

## Description

### Field of the invention

The present invention relates to a cosmetic use of a phenyl-butyric acid, a derivative or a physiologically acceptable salt thereof, like 4-phenylbutyric acid (4-PB) or its sodium salt. Particularly, the present invention provides cosmetic articles comprising phenyl-butyric acid, a derivative or a physiologically acceptable salt thereof, like 4-phenylbutyric acid (4-PB) or its sodium salt, especially at a low daily dosis for cosmetic use for treatment of various cosmetic problems of body surfaces such as hair, skin etc..

### Background of the invention:

Even though not life threatening, cosmetic problems can have a high impact on the quality of life. There are numerous products on the market for treating these cosmetic problems including varicosis, skin discolouration, thin hair etc. The success is often very limited or not forthcoming.

Accordingly, it was the objective of the present invention to provide a new form of cosmetic treatment

4-Phenylbutyric acid (4-PB) is a low molecular weight aromatic carboxylic acid.

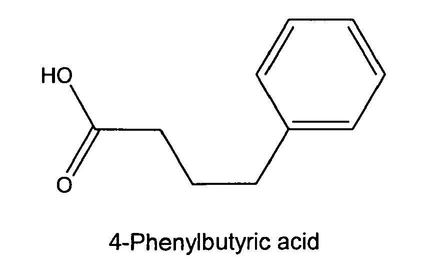

"4-Phenylbutyric acid" (abbreviated as "4-PB") is defined herein as encompassing not only 4-Phenybutyric acid, as free acid but also its derivatives, and physiologically acceptable salts thereof. Especially, "4-Phenylbutyric acid" or "4-PB" is also defined as its free acid, but also as being in the form of a pharmaceutically acceptable salt, co-crystal, polymorph, hydrate, solvate or pro-drug of 4-phenylbutyric acid. Most preferably, "4-Phenylbutyric acid" or "4-PB" is either the free acid or a pharmaceutically acceptable salt of 4-PB, such as its sodium salt. This definition is to be applied in all cases within this description or the attached claims in which the expression "4-Phenylbutyric acid" or "4-PB" is used unless being more precisely defined as e.g. "4-Phenylbutyric acid as free acid" or "sodium salt of 4-PB".

The 4-phenylbutyric acid sodium salt (sodium 4-phenylbutyrate) and its use for the treatment of various clinical conditions and diseases such as benign prostate hyperplasy, cancer, cystic fibrosis, HIV, kidney and liver failure, thalassemia and especially urea cycle disorders for which it is clinically used is well known.

### Summary of the invention

The present inventors have surprisingly found that 4-PB can be used with very good effects as a solution for various cosmetical problems like treatment of varicosis even in the peripheral vein or in hair growth, especially when administered at low dosis.

Based on the above findings, the present inventors provide the following aspects which summarize the present invention:

One aspect of the invention relates to 4-phenylbutyric acid (4-PB) for use as a cosmetic. Preferably, 4-PB is administered to a subject in a daily amount of at most about 500 mg.

In principle, this invention covers the use of 4-phenylbutyric acid (4-PB) as or in a cosmeceutical or as a nutricosmetic. At the base of this invention lies the finding that 4-PB even though biologically active can be also with success in cosmetic applications, especially in dose ranges far below the ranges for which 4-PB received marketing authorization as a pharmaceutical.

The use of this cosmetic uses range from the treatment of a body surface like hair, lip, eye, or skin with the intention of achieving an aesthetic effect; also covering the treatment of greying hair, varicosis, especially peripheral varicosis, hair loss, optical changes of the skin, hair and nails, as well as the treatment for achieving hair growth, nail and hair stability, clearness of skin and anti aging effects like removing of wrinkles

Another aspect of the invention relates to a cosmetic article, comprising 4-phenylbutyric acid (4-PB), preferably in an amount of at most about 500mg optionally with one or more excipients. This cosmetic article can either be adapted for oral or dermal application, preferably being a sustained release formulation for oral administration or dermal application and/or is in form of either capsules or tablets or of lotions, creams or ointments.

Having summarized the various aspects of the present invention, the invention is now described in more detail. It is intended that each and every embodiment described hereafter may form part of any one of the above aspects. Moreover, it is explicitly intended that the teaching of this invention covers any combination of these embodiments.

### Detailed description of the invention

The present invention provides 4-phenylbutyric acid (4-PB) for use as a cosmetic.

"Cosmetic" is defined herein as a compound or formulation of compounds or device which is/are used for achieving an ameliorating aesthetic effect mostly on a human being. "Cosmetic" in the narrower sense of this invention is meant to also include a "cosmeceutical" (and also "nutricosmetics") a cosmetic comprising a biologically active compound, in this case 4-PB. Cosmetics may often take the form of a topical/dermal formulation such as ointments, creams or lotions but can also be capsules or tablets.

"Cosmeceutical" is a cosmetic comprising a biologically active compound, in this case 4-PB. "Cosmeceuticals" will most often take the form of a topical/dermal formulation such as ointments, creams or lotions but can also be capsules or tablets. In the narrower sense it is understood that "Cosmeceuticals" come in the form of a topical/dermal formulation such as ointments, creams or lotions.

"Nutricosmetics" is a cosmetic also comprising a biologically active compound, in this case 4-PB. "Nutricosmetics" are in orally applicable form such as capsules or tablets.

All of them, "Cosmetics", "Cosmeceutical", and "Nutricosmetics" have in common that the biologically active compound, in this case 4-PB is applied and used in dose ranges far below the ranges for which 4-PB received marketing authorization as a pharmaceutical.

According to some embodiments, the invention provides 4-phenylbutyric acid (4-PB) for use according to the invention wherein 4-PB is administered to a subject in a daily amount of at most about 500 mg.

According to some embodiments, the invention provides 4-phenylbutyric acid (4-PB) for use according to the invention wherein the daily amount of 4-PB to be administered is at most about 450 mg, at most about 400 mg, at most about 350 mg, at most about 300 mg, at most about 250 mg, at most about 200 mg, at most about 150 mg, at most about 100 mg, at most about 75 mg or at most about 50 mg or at most about 25 mg.

According to some embodiments, the invention provides 4-phenylbutyric acid (4-PB) for use according to the invention, wherein the daily amount of 4-PB, derivative or physiologically acceptable salt thereof, to be administered is at most about 250 mg
and/or
wherein the daily amount of 4-PB, derivative or physiologically acceptable salt thereof, to be administered is at least about 50 mg or 25 mg.

According to some embodiments, 4-PB is administered to a subject in a daily amount of at most about 500 mg.

According to some embodiments, the daily amount of 4-PB to be administered may be at most about 450 mg, at most about 400 mg, at most about 350 mg, at most about 300 mg, at most about 250 mg, at most about 200 mg, at most about 150 mg, at most about 100 mg, at most about 75 mg or at most about 50 mg or at most about 25 mg.

According to some embodiments, the daily amount of 4-PB to be administered is at most about 100 mg

According to some embodiments, the daily amount of 4-PB to be administered is at most about 250 mg.

According to some embodiments, the daily amount of 4-PB to be administered is at most about 500 mg.

According to some embodiments, the daily amount of 4-PB to be administered is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, or at least about 500 mg.

According to some embodiments, the daily amount of 4-PB to be administered is at least about 25 mg.

According to some embodiments, the daily amount of 4-PB to be administered is at least about 50 mg.

According to some embodiments, the daily amount of 4-PB to be administered is at least about 75 mg.

According to some embodiments, the daily amount of 4-PB to be administered is at least about 100 mg.

According to some embodiments, the daily amount of 4-PB to be administered is at least about 250 mg.

According to some embodiments, the daily amount of 4-PB to be administered is at least about 500 mg.

According to some embodiments, the daily amount of 4-PB to be administered is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the daily amount of 4-PB to be administered is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the daily amount of 4-PB to be administered is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, 4-PB is administered one or more times a day, such as, e.g., two times, three times, four times or five times. According to some embodiments, 4-PB is administered once a day. According to some embodiments, 4-PB is administered twice a day. According to some embodiments, 4-PB is administered once in the morning and once in the evening.

According to some embodiments, 4-PB is administered two times or more a day, such as, e.g., three times, four times or five times, wherein the administrations are 2 to 14 hours apart, such as, e.g., 4, 6, 8, 10 or 12 hours apart. According to specific embodiments, 4-PB is administered twice a day, wherein the administrations are 8 to 12 hours apart.

According to some embodiments, 4-PB is administered in form of a pharmaceutically acceptable salt, co-crystal, polymorph, hydrate, solvate or pro-drug. The phrase "pharmaceutically acceptable", as used herein, means those salts, co-crystals, polymorphs, hydrates, solvates and pro-drugs of 4-PB that are safe in terms of toxicity, irritation, allergic response, or other problem or compliance, and effective for therapeutic use in mammals, in particular humans.

Pharmaceutically acceptable salts of 4-PB are known in the art and include salts derived from inorganic or organic acids. A pharmaceutically acceptable salt of 4-PB suitable for use in the present invention can be an alkali metal salt (e.g. sodium), an earth alkali metal salt, an ammonium salt, a substituted ammonium salt, or an acid addition salt.

The alkali metal salts can be prepared from the free acid by means well known to those of skill in the art (e.g., reaction of the free carboxylic acid with an alkali metal hydroxide or alkoxide in an appropriate solvent). The acid addition salts of 4-PB suitable can be generated from the free-base forms of the compounds by reaction of the latter with one equivalent of a suitable, non-toxic, pharmaceutically-acceptable acid, followed by evaporation of the solvent employed for the reaction and recrystallization of the salt, as required. Suitable acids for forming acid addition salts of 4-PB include, but are not limited to, acetic, benzoic, benzenesulfonic, tartaric, hydrobromic, hydrochloric, citric, fumaric, gluconic, glucuronic, glutamic, lactic, malic, maleic, methanesulfonic, palmoic, salicylic, stearic, succinic, sulfuric, and tartaric acids. The class of acids suitable for formation of nontoxic, pharmaceutically-acceptable salts is well known to the persons skilled in the art, and are described, for example in Stahl, P.H., et al., "Handbook of Pharmaceutical Salts", Wiley-VCH, Weinheim: Germany (2002). The free base of 4-PB, if required, can be recovered from an acid addition salt thereof by reaction of the salt with a water solution of the salt with a suitable base such as sodium carbonate, sodium hydroxide, and the like.

The salts can be prepared in situ during the final isolation and purification of the compounds of the present invention or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared in situ during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammoniurn, tetraethylammonium, methylamine, dimethylamine, trimethylarnine, triethylamine, diethylamine, ethylamine and the like. Other representative organic amines useful for the formation of base addition salts include ethylenediamine, ethanolamine, diethanolamine, piperidine, piperazine and the like. Salts of 4-PB also include phosphate, tris and acetate.

Pharmaceutically acceptable salts may be also obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium or magnesium) salts of carboxylic acids can also be made.

According to some embodiments, the 4-PB salt is an alkali metal salt selected from the group of lithium, sodium, potassium, calcium, magnesium, aluminium and cesium.

According to some embodiments, the 4-PB salt is the sodium salt, sodium 4-phenylbutyrate.

A co-crystal of 4-PB, as referred to herein, is a crystalline material comprising two or more compounds at ambient temperature (20 to 25°C, in particular 20°C), of which at least two are held together by weak interaction, wherein at least one of the compounds is a co-crystal former. Weak interaction is being defined as an interaction, which is neither ionic nor covalent and includes for example: hydrogen bonds and van der Waals forces. Solvates or salts of phenyl butyric acid and an organic compound that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself. See also Zaworotko (Zaworotko, Crystal Growth & Design, Vol. 7, No. 1, 2007, 4-9) which gives a definition of co-crystal which is in line with the definition given above.

Polymorphs of 4-PB, as referred to herein, are crystalline forms of 4-PB having the same chemical composition but different spatial arrangements of the molecules, atoms, and/or ions forming the crystal. "Polymorphs" in general are described in "Polymorphism in Pharmaceutical Solids", Harry G. Brittain (ed.) Drugs and the Pharmaceutical Sciences 192 (1999).

A solvate of 4-PB, as referred to herein, is a physical association of 4-PB with one or more solvent molecules, whether organic or inorganic. This physical association includes hydrogen bonding. In certain instances the solvate will be capable of isolation, for example when one or more solvent molecules are incorporated in the crystal lattice of the crystalline solid. The solvate may comprise either a Stoichiometric or nonstoichiometric amount of the solvent molecules. For example, a solvate with a nonstoichiometric amount of solvent molecules may result from partial loss of solvent from the solvate. "Solvate" encompasses both solution-phase and isolable solvates. Exemplary solvates include hydrates, ethanolates, methanolates, isopropanolates and the like. Methods of solvation are generally known in the art.

A hydrate of 4-PB, as referred to herein, is a substance containing 4-PB and water. It may be a crystalline salt with one or more water molecules in the crystalline structure or also amorphous containing water molecules.

A prodrug of 4-PB is a compound that is transformed in vivo to 4-PB, for example, by hydrolysis. Prodrug design is discussed generally in Hardma et al. (Eds.), Goodman and Gilman's The Pharmacological Basis of Therapeutics, 9th ed., pp. 11-16 (1996). Another discussion is also provided by Higuchi, et al., in Prodrugs as Novel Delivery Systems. Vol. 14. ASCD Symposium Series, and in Roche (ed.), Bioreversible Carriers in Drug Design. American Pharmaceutical Association and Pergamon Press (1987). Typically, administration of a drug is followed by elimination from the body or some biotransformation whereby the biological activity of the drug is reduced or eliminated. Alternatively, a biotransformation process can lead to a metabolic by-product that is more or equally active compared to the drug initially administered. Increased understanding of these biotransformation processes permits the design of so-called "prodrugs," which, following a biotransformation, become more physiologically active in their altered state. Prodrugs, therefore, as used within the scope of the present disclosure, encompass compounds that are converted by some means to pharmacologically active metabolites.

According to some embodiments, 4-PB is administered in combination with one or more amino acids (such as, e.g. two, three, four, five, six, seven, eight, nine or ten amino acids). The one or more amino acids may be proteinogenic and/or non-proteinogenic. Proteinogenic amino acids, as referred to herein, are those amino acids that can be found in proteins and require cellular machinery coded for in the genetic code of any organism for their isolated production. Proteinogenic amino acids thus include the 22 standard amino acids. Non-proteinogenic amino acids, as referred to herein, are those amino acids which are either not found in proteins, or are not produced directly and in isolation by standard cellular machinery.

According to some embodiments, the one or more amino acids are L-amino acids or D-amino acids, or a combination thereof.

According to specific embodiments, the one or more amino acids is/are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According to some embodiments, the one or more amino acids is/are selected from alanine, valine, leucine, isoleucine, proline, methionine, phenylalanine and tryptophane.

According to some embodiments, the one or more amino acids is/are selected from serine, threonine, asparagine, glutamine, cysteine and tyrosine.

According to some embodiments, the one or more amino acids is/are selected from aspartic acid and glutamic acid.

According to some embodiments, the one or more amino acids is/are selected from lysine, arginine and histidine.

According to some embodiments, the one or more amino acids is/are selected from alanine, valine, Leucine and isoleucine.

According to some embodiments, the one or more amino acids is/are selected from cysteine and methionine.

According to some embodiments, the one or more amino acid is/are selected from phenylalanine, tyrosine and tryptophane.

According to some embodiments, the one or more amino acid is/are selected from serine, threonine, asparagine and glutamic acid.

According to some embodiments, the one or more amino acid is/are selected from selenocysteine, selenomethionine, norleucine, norvaline, ornithine, penicillamine, hydroxyproline, homoserine, homophenylalanine, homoleucine, homonorleucine and homoarginine,

According to some embodiments, the one or more amino acid is selected from alanine, arginine, aspargine, aspartic acid, cysteine, glutamic acid, glutamine, histidine, lsoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According to some embodiments, the one amino acid is alanine, especially D-alanine.

According to some embodiments, the one or more amino acids is/are not carnitine, homocystein and canavanine, neither in the L- nor in the D-configuration.

According to some embodiments, the amount of the one or more amino acids to be administered in combination with 4-PB is in the range from about 1:10 to about 10:1 w/w of the amount of 4-PB, each or total.

According to some embodiments, the amount of the one or more amino acids to be administered in combination with 4-PB is in the range from about 1:5 to about 5:1 w/w of the amount of 4-PB, each or total.

According to some embodiments, the amount of the one or more amino acids to be administered in combination with 4-PB is in the range from about 1:2 to about 2:1 w/w of the amount of 4-PB, each or total.

According to some embodiments, the amount of the one or more amino acids to be administered in combination with 4-PB is 1:1 w/w of the amount of 4-PB, each or total.

According to some embodiments, use as a cosmetic of the 4-phenylbutryic acid for use according to the invention is a use for the treatment of a body surface like hair, lip, eye, or skin with the intention of achieving an aesthetic effect; preferably is the treatment of greying hair, varicosis, especially peripheral varicosis, hair loss, optical changes of the skin, hair and nails, as well as the treatment for achieving hair growth, nail and hair stability, clearness of skin and anti aging effects like removal or ameliorating of wrinkles and/or tightening the skin.

According to some embodiments, one proviso applies that the use as a cosmetic does not cover treatment of hair loss.

According to some embodiments, another proviso applies that the use as a cosmetic does not cover treatment of grey hair.

According to some embodiments, another proviso applies that the use as a cosmetic does not cover treatment of varicosis.

According to some embodiments, another proviso applies that one some or all of the the cosmetic uses are not a disease, disorder or symptom caused by a genetic disorder or epigenetic disorder, thus are not a disease, disorder or symptom whose cause rests in a genetic disorder, especially in an epigenetic disorder.

"*Genetic disorder*" is defined as disorders that are caused by and/or based on changes in one or more genes that are inherited from at least one of the parents. Examples include urea cycle disorders, thalassemia, but also the embodiments of diseases or symptoms such as varicosis, vaginitis, depression or Sudden Infant Death Syndrome etc., that are based on or caused by these changes. A list of relevant disorders or related diseases and symptoms follows below.

"*Epigenetic" or "epigenetic disorder"* is defined as an inherited change in phenotype or gene expression which is not caused by changes to the gene sequence but is caused other mechanism/non-genetic factors.

According to some embodiments, 4-PB is administered by oral or dermal application, preferably as a sustained release by oral administration or dermal application and or in form of either capsules or tablets or lotions, creams or ointments.

By "sustained release" (of the active ingredient = 4-PB) is to be understood especially a rate of release of the active ingredient during a period of about 6 to 12 or up to 24 hours.

According to some embodiments, 4-PB is administered by oral application, in some cases as a sustained release.

According to some embodiments, 4-PB is administered by oral application in form of a tablet or capsule adapted to release the dose ranges given above.

According to some embodiments, 4-PB is administered by dermal/topical application, in some cases as a sustained release.

According to some embodiments, 4-PB is administered by dermal/topical application in form of lotions, creams or ointments. In some embodiments these topical formulations (lotions, creams or ointments) comprise about 1 to 10% (w/v), 2 to 8% (w/v), 3 to 7% (w/v), 4 to 6% (w/v) or about 5% (w/v) of 4-PB.

The present invention further provides a cosmetic article comprising 4-phenylbutyric acid (4-PB).

"Cosmetic article" is defined herein as a device which is used for achieving an ameliorating aesthetic effect mostly on a human being. "Cosmetic article" in the narrower sense of this invention is meant to also include a "cosmeceutical" (and also "nutricosmetics") a cosmetic comprising a biologically active compound, in this case 4-PB. Cosmetic articles may often take the form of a topical/dermal formulation such as ointments, creams or lotions but can also be capsules or tablets.

"Cosmeceutical" is already defined above and is a cosmetic comprising a biologically active compound, in this case 4-PB. "Cosmeceuticals" will most often take the form of a topical/dermal formulation such as ointments, creams or lotions but can also be capsules or tablets. In the narrower sense it is understood that "Cosmeceuticals" come in the form of a topical/dermal formulation such as ointments, creams or lotions.

"Nutricosmetics" is already defined above and is a cosmetic also comprising a biologically active compound, in this case 4-PB. "Nutricosmetics" are in orally applicable form such as capsules or tablets.

All of them, "Cosmetic article", "Cosmeceutical", and "Nutricosmetics" have in common that the biologically active compound comprised in them, in this case 4-PB, is applied and used in dose ranges far below the ranges for which 4-PB received marketing authorization as a pharmaceutical.

According to some embodiments, the invention provides a cosmetic article according to the invention comprising 4-phenylbutyric acid in an amount of at most about 500 mg and optionally one or more pharmaceutically acceptable carriers, diluents and/or excipients.

According to some embodiments, the invention provides a cosmetic article according to the invention, wherein the amount of 4-PB is at most about 450 mg, at most about 400 mg, at most about 350 mg, at most about 300 mg, at most about 250 mg, at most about 200 mg, at most about 150 mg, at most about 100 mg, at most about 75 mg or at most about 50 mg or at most about 25 mg.

According to some embodiments, the invention provides a cosmetic article according to the invention, wherein the amount of 4-PB is at most about 250 mg
and/or
wherein the amount of 4-PB is at least about 25 mg or 50 mg.

According to the invention, the cosmetic article comprises 4-PB in an amount of at most about 500 mg and one or more pharmaceutically acceptable carriers, diluents and/or excipients.

The cosmetic article according to the invention can be of any form suitable for the application to humans and/or animals, preferably humans including infants, children and adults and can be produced by standard procedures known to those skilled in the art.

According to some embodiments, the cosmetic article according to the invention is adapted for oral or dermal application, preferably is a sustained release formulation for oral administration or dermal/topical application and/or is in form of either capsules or tablets or of lotions, creams or ointments.

By "sustained release" (of the active ingredient = 4-PB) is to be understood especially a rate of release of the active ingredient during a period of about 6 to 12 or up to 24 hours.

According to some embodiments, the cosmetic article according to the invention is adapted for oral application, in some cases is a sustained release formulation for oral administration.

According to some embodiments, the cosmetic article according to the invention is in form of either capsules or tablets adapted to release the dose ranges given above

According to some embodiments, the cosmetic article according to the invention is adapted for dermal/topical application, in some cases is a sustained release formulation for oral administration.

According to some embodiments, the cosmetic article according to the invention is in form of lotions, creams or ointments. In some embodiments these topical formulations (lotions, creams or ointments) comprise about 1 to 10% (w/v), 2 to 8% (w/v), 3 to 7% (w/v), 4 to 6% (w/v) or about 5% (w/v) of 4-PB.

Carriers, diluents and excipients which are suitable for the preparation of a cosmetic article according to the present invention are well known to those skilled in the art.

According to some embodiments, the amount of 4-PB in the cosmetic article is at most about 500 mg, at most about 450 mg, at most about 400 mg, at most about 350 mg, at most about 300 mg, at most about 250 mg, at most about 200 mg, at most about 150 mg, at most about 100 mg, at most about 75 mg, at most about 50 mg, or at most about 25 mg.

According to some embodiments, the amount of 4-PB in the cosmetic article is at most about 100 mg

According to some embodiments, the amount of 4-PB in the cosmetic article is at most about 250 mg.

According to some embodiments, the amount of 4-PB in the cosmetic article is at most about 500 mg.

According to some embodiments, the amount of 4-PB in the cosmetic article is at least about 25 mg, at least about 50 mg, at least about 75 mg, at least about 100 mg, at least about 150 mg, at least about 200 mg, at least about 250 mg, at least about 300 mg, at least about 350, at least about 400 mg, at least about 450 mg, or at least about 500 mg.

According to some embodiments, the amount of 4-PB in the cosmetic article is at least about 25 mg.

According to some embodiments, the amount of 4-PB in the cosmetic article is at least about 50 mg.

According to some embodiments, the amount of 4-PB in the cosmetic article is at least about 75 mg.

According to some embodiments, the amount of 4-PB in the cosmetic article is at least about 100 mg.

According to some embodiments, the amount of 4-PB in the cosmetic article is at least about 250 mg.

According to some embodiments, the amount of 4-PB in the cosmetic article is at least about 500 mg.

According to some embodiments, the amount of 4-PB in the cosmetic article is in the range from about 25 mg to about 500 mg, from about 50 mg to about 500 mg, from about 75 mg to about 500 mg, from about 100 to about 500 mg, from about 150 to about 500 mg, from about 200 mg to about 500 mg, from about 250 mg to about 500 mg, from about 300 mg to about 500 mg, from about 350 mg to about 500 mg, from about 400 mg to about 500 mg or from about 450 to about 500 mg.

According to some embodiments, the amount of 4-PB in the cosmetic article is in the range from about 25 mg to about 250 mg, from about 50 mg to about 250 mg, from about 75 mg to about 250 mg, from about 100 to about 250 mg, from about 125 mg to about 250 mg, from about 150 to about 250 mg, from about 175 mg to about 250 mg or from about 200 mg to about 250 mg.

According to some embodiments, the amount of 4-PB in the cosmetic article is in the range from about 25 mg to about 100 mg, from about 50 mg to about 100 mg, from about 55 mg to about 100 mg, from about 60 mg to about 100 mg, from about 65 mg to about 100 mg, from about 70 mg to about 100 mg, from about 75 mg to about 100 mg, from about 80 mg to about 100 mg, from about 85 mg to about 100 mg, from about 90 mg to about 100 mg or from about 95 to about 100 mg.

According to some embodiments, the cosmetic article comprises 4-PB in the form of a pharmaceutically acceptable salt, co-crystal, polymorph, hydrate, solvate or pro-drug. For further details on pharmaceutically acceptable salts, co-crystals, polymorphs, hydrates, solvates and pro-drugs of 4-PB reference is made to above description.

According to some embodiments, the 4-PB salt is an alkali metal salt selected from the group of lithium, sodium, potassium, calcium, magnesium, aluminium and cesium.

According to some embodiments, the 4-PB salt is the sodium salt, sodium 4-phenylbutyrate.

According to some embodiments, the cosmetic article further comprises one or more amino acids (such as, e.g. two, three, four, five, six, seven, eight, nine or ten amino acids).

According to some embodiments, the one or more amino acids is/are L-amino acid(s) or D-amino acid(s), or a combination thereof.

According to specific embodiments, the one or more amino acids is/ are in the D-configuration.

According to some embodiments, the one or more amino acids is/are selected from nonpolar, polar, acidic or basic amino acids.

According to some embodiments, the one or more amino acids is/are selected from aliphatic, sulfur-containing, aromatic or neutral amino acids.

According some embodiments, the one or more amino acids is/are selected from alanine, valine, leucine, isoleucine, proline, methionine, phenylalanine and tryptophane.

According some embodiments, the one or more amino acids is/are selected from serine, threonine, asparagine, glutamine, cysteine and tyrosine.

According some embodiments, the one or more amino acids is/are selected from aspartic acid and glutamic acid.

According some embodiments, the one or more amino acids is/are selected from lysine, arginine and histidine.

According some embodiments, the one or more amino acids is/are selected from alanine, valine, Leucine and isoleucine.

According some embodiments, the one or more amino acids is/are selected from cysteine and methionine.

According some embodiments, the one or more amino acid is/are selected from phenylalanine, tyrosine and tryptophane.

According some embodiments, the one or more amino acid is/are selected from serine, threonine, asparagine and glutamic acid.

According some embodiments, the one or more amino acid is/are selected from selenocysteine, selenomethionine, norleucine, norvaline, ornithine, penicillamine, hydroxyproline, homoserine, homophenylalanine, homoleucine, homonorleucine and homoarginine,

According some embodiments, the one or more amino acid is selected from alanine, arginine, aspargine, aspartic acid, cysteine, glutamic acid, glutamine, histidine, lsoleucine, leucine, lysine, methionine, phenylalanine, proline, serine, threonine, tryptophane, tyrosine, valine, selenocysteine, selenomethionine, norleucine, norvaline, ornithine, penicillamine, hydroxyproline, homoserine, homophenylalanine, homonorleucine and homoarginine.

According some embodiments, the one amino acid is alanine, especially D-alanine.

According some embodiments, the one or more amino acids is/are not carnitine, homocystein and canavanine, neither in the L- nor in the D-configuration.

According to some embodiments, the amount of the one or more amino acids in the cosmetic article is in the range from about 1:10 to about 10:1 w/w of the amount of 4-PB, each or total.

According to some embodiments, the amount of the one or more amino acids in the cosmetic article is in the range from about 1:5 to about 5:1 w/w of the amount of 4-PB, each or total.

According to some embodiments, the amount of the one or more amino acids in the cosmetic article is in the range from about 1:2 to about 2:1 w/w of the amount of 4-PB, each or total.

According to some embodiments, the amount of the one or more amino acids in the cosmetic article is about 1:1 w/w of the amount of 4-PB, each or total.

According to some embodiments, the cosmetic article according to the invention is administered one or more times a day, such as, e.g., two times, three times, four times or five times. According to some embodiments, the cosmetic article according to the invention is administered once a day. According to some embodiments, the cosmetic article according to the invention is administered twice a day. According to some embodiments, the cosmetic article according to the invention is administered once in the morning and once in the evening.

According to some embodiments, the cosmetic article according to the invention is administered two times or more a day, such as, e.g., three times, four times or five times, wherein the administrations are 2 to 14 hours apart, such as, e.g., 4, 6, 8, 10 or 12 hours apart. According to specific embodiments, the cosmetic article according to the invention is administered twice a day, wherein the administrations are 8 to 12 hours apart.

According to some embodiments, the cosmetic article according to the invention is in form of a sustained release formulation, preferably is in form of an oral sustained release formulation.

By sustained release of the active ingredient (being 4-PB) is to be understood especially a rate of release of the active ingredient (here 4-PB) during a period of about 6 to 12 or up to 24 hours.

According to some embodiments, the cosmetic article according to the invention is for use in the treatment of a body surface like hair, lip, eye, or skin with the intention of achieving an aesthetic effect; preferably for use in the treatment of greying hair, varicosis, especially peripheral varicosis, hair loss, optical changes of the skin, hair and nails, as well as for use in treatment for achieving hair growth, nail and hair stability, clearness of skin and anti aging effects like removal or ameliorating of wrinkles and/or tightening the skin.

According to some embodiments, one proviso applies that the use of the cosmetic article according to the invention does not cover treatment of hair loss.

According to some embodiments, one proviso applies that the use of the cosmetic article according to the invention does not cover treatment of grey hair.

According to some embodiments, one proviso applies that the use of the cosmetic article according to the invention does not cover treatment of varicosis.

According to some embodiments, another proviso applies that one some or all of the uses of the cosmetic article according to the invention are not treatment of a disease, disorder or symptom caused by a genetic disorder or epigenetic disorder, thus are not treatment of a disease, disorder or symptom whose cause rests in a genetic disorder, especially in an epigenetic disorder.

*"Genetic disorder"* is defined as disorders that are caused by and/or based on changes in one or more genes that are inherited from at least one of the parents. Examples include urea cycle disorders, thalassemia, but also the embodiments of diseases or symptoms such as varicosis, vaginitis, depression or Sudden Infant Death Syndrome etc., that are based on or caused by these changes. A list of relevant disorders or related diseases and symptoms follows below.

*"Epigenetic" or "epigenetic disorder"* is defined as an inherited change in phenotype or gene expression which is not caused by changes to the gene sequence but is caused other mechanism/non-genetic factors.

It is again explicitly intended that the teaching of this invention covers any combination of the above described embodiments.

The present invention is illustrated below with the help of the following figures and examples. These illustrations are given solely by way of example and do not limit the invention.

### Examples

### Example 1: Production of slow release tablet with 250 mg of sodium 4-phenylbutyrate

A mixture of 6,000.0 g of sodium 4-phenylbutyrate from Triple Crown America, Inc., 6,280.0 g of lactosum monohydriculum, 3,500.0 g of Methocel K100 M Preium (Prochem), and 750.0 g of Avicel PH 102 (Select Chemie) is wettened with 4,000.0 g of aqua purificata (water purified by inversion osmosis) and dried in cold air during 10 hours with air of 40°C. A mixture of 240.0 g of talcum and 30.0 g of magnesium stearate is admixed during 20 minutes and the mixture is pressed into tablets of 0.70 g each, a thickness of about 6.8 mm and with a hardness of 90 Newton. Yield: 24,000 tablet cores.

The mixing is carried out with a Diosna Mixer, the drying in a Lükon drying cabinet, the sieving with a Köhler & Bosshard sieving machine, and the tablet pressing with a Korsch tablet press EK 11.

The cores are provided with a film coating for example by using a colloidal dispersion containing 7,850 g of isopropylalcohol, 3,360 g of Eudragit L 12.5, 66 g of dibutyl phthalat, 18.0 g of Miglyol 812, and 56 g of polyethylenglycol PEG 400. The suspension is sprayed at 3.5 atü and 25° C onto the 24,000 cores. The film-coated tablets are dried in a circulating air drying cabinet for at least 4 hours at 35°C.

## Claims

1. 4-phenylbutyric acid (4-PB) for use as a cosmetic.

2. 4-phenylbutyric acid for use according to claim 1, wherein 4-PB is administered to a subject in a daily amount of at most about 500 mg, preferably wherein the daily amount of 4-PB to be administered is at most about 450 mg, at most about 400 mg, at most about 350 mg, at most about 300 mg, at most about 250 mg, at most about 200 mg, at most about 150 mg, at most about 100 mg, at most about 75 mg or at most about 50 mg or at most about 25 mg.

3. 4-phenylbutyric acid for use according to claim 1 or 2, wherein the daily amount of 4-PB, derivative or physiologically acceptable salt thereof, to be administered is at most about 250 mg
and/or
wherein the daily amount of 4-PB, derivative or physiologically acceptable salt thereof, to be administered is at least about 50 mg or 25 mg.

4. 4-phenylbutryic acid for use according to any one of claims 1 to 3, wherein 4-PB is in the form of a pharmaceutically acceptable salt, co-crystal, polymorph, hydrate, solvate or pro-drug.

5. 4-phenylbutryic acid for use according to any one of claims 1 to 4, wherein the use as a cosmetic is a use for the treatment of a body surface like hair, lip, eye, or skin with the intention of achieving an aesthetic effect; preferably is the treatment of greying hair, varicosis, especially peripheral varicosis, hair loss, optical changes of the skin, hair and nails, as well as the treatment for achieving hair growth, nail and hair stability, clearness of skin and anti aging effects like removal or ameliorating of wrinkles and/or tightening the skin.

6. 4-phenylbutryic acid for use according to any one of claims 1 to 7, wherein 4-PB is administered by oral or dermal application, preferably as a sustained release by oral administration or dermal application and or in form of either capsules or tablets or lotions, creams or ointments.

7. A Cosmetic article comprising 4-phenylbutyric acid (4-PB).

8. The Cosmetic article according to claim 7, comprising 4-phenylbutyric acid in an amount of at most about 500 mg and one or more pharmaceutically acceptable carriers, diluents and/or excipients.

9. The Cosmetic article according to claims 7 or 8, wherein the amount of 4-PB is at most about 450 mg, at most about 400 mg, at most about 350 mg, at most about 300 mg, at most about 250 mg, at most about 200 mg, at most about 150 mg, at most about 100 mg, at most about 75 mg or at most about 50 mg or at most about 25 mg.

10. The cosmetic article for use according to any of claim 7 to 9, wherein the amount of 4-PB is at most about 250 mg
and/or
wherein the amount of 4-PB is at least about 25 mg or 50 mg.

11. The Cosmetic article according to any of claims 7 to 10, for use in the treatment of a body surface like hair, lip, eye, or skin with the intention of achieving an aesthetic effect; preferably for use in the treatment of greying hair, varicosis, especially peripheral varicosis, hair loss, optical changes of the skin, hair and nails, as well as for use in treatment for achieving hair growth, nail and hair stability, clearness of skin and anti aging effects like removal or ameliorating of wrinkles and/or tightening the skin.

12. The cosmetic article according to any one of claims 7 to 11, wherein 4-PB is in the form of a pharmaceutically acceptable salt, co-crystal, polymorph, hydrate, solvate or pro-drug.

13. The pharmaceutical composition for use according to any one of claims 7 to 12, further comprising one or more amino acid(s), such as, e.g., D-amino acid(s).

14. The cosmetic article according to any one of claims 7 to 13, wherein the cosmetic article is adapted for oral or dermal application, preferably is a sustained release formulation for oral administration or dermal application and/or is in form of either capsules or tablets or of lotions, creams or ointments.
